# EUROPÄISCHE PATENTSCHRIFT

(11) **EP 2 229 950 B1**
(45) Veröffentlichungstag und Bekanntmachung des Hinweises auf die Patenterteilung: **22.06.2011**
(21) Anmeldenummer: 10002550.1
(22) Anmeldetag: 11.03.2010
(51) Int. Cl.: A61K 36/53, A61P 25/00

(54) **Verwendung von Pflanzen der Gattung Sideritis und Extrakten daraus zur Behandlung des Aufmerksamkeitsdefizit-Hyperaktivitätssyndroms**
Use of plants of the sideritis genus and extracts from same for treating attention deficit/hyperactivity disorder
Utilisation de plantes du genre Sideritis et extraits de celles-ci pour le traitement du syndrome du déficit de l'attention avec hyperactivité

(30) Priorität: 17.03.2009 DE 102009013225
(43) Veröffentlichungstag der Anmeldung: 22.09.2010
(73) Patentinhaber: IBAM GbR, 79211 Denzlingen (DE)
(72) Erfinder: Knörle, Rainer, Dr., 79263 Simonswald (DE); Schnierle, Peter, Dr., 79114 Freiburg (DE)
(74) Vertreter: Stoppkotte, Cornelia

(56) Entgegenhaltungen:
- ESPLUGUES J ET AL: "ACTIVITE PHARMACODYNAMIQUE DE SIDERITIS MUGRONENSIS.1. TOXICITE ET ACTION SUR LE S.N.C.//PHARMACODYNAMIC ACTIVITY OF SIDERITIS MUGRONENSIS. 1. TOXICITY AND ACTION ON THE CNS" PLANTES MEDICINALES ET PHYTOTHERAPIE, ANGERS, FR, Bd. 16, Nr. 2, 1. Januar 1982 (1982-01-01) , Seiten 137-146, XP009060468 ISSN: 0032-0994
- Rainer Knörle, Peter Schnierle: "Extrakte aus Sideritis ssp. (griechischer Bergtee): Innovative zentral aktive Pflanzenextrakte mit breitem Wirkprofil" 1. Oktober 2009 (2009-10-01), Seiten 1-15, XP002591208 Gefunden im Internet: URL:http://ibam.de/pics/Poster-Wolnzach-20 09.pdf [gefunden am 2010-07-08]

## Beschreibung

Die vorliegende Erfindung betrifft die Verwendung von Pflanzen der Gattung *Sideritis* und/oder deren Pflanzenteilen und/oder deren Extrakten zur Behandlung des Aufinerksamkeitsdefizit-Hyperaktivitätssyndroms.

Das Aufmerksamkeitsdefizit-Hyperaktivitätssyndrom (ADHS), das auch als Aufmerksamkeitsdefizit-Hyperaktivitätsstörung oder Hyperkinetische Störung bezeichnet wird, ist eine bereits im Kindesalter beginnende psychische Störung, die sich durch Probleme mit der Aufmerksamkeit sowie Impulsivität und häufig auch Hyperaktivität auszeichnet. Etwa drei bis zehn Prozent aller Kinder zeigen Symptome im Sinne einer ADHS. Jungen sind deutlich häufiger betroffen als Mädchen. Die Symptome können mit unterschiedlicher Ausprägung bis in das Erwachsenenalter hinein fortbestehen.

Es existieren weiterhin alternative Bezeichnungen und Abkürzungen, welche teilweise übereinstimmende Krankheitsbilder beschreiben, teilweise spezielle Ausprägungen bezeichnen. Verbreitet ist besonders die Bezeichnung Aufmerksamkeitsdefizitstörung (ADS). Veraltet sind hingegen die Bezeichnungen Minimale Cerebrale Dysfunktion (MCD) und Psychoorganisches Syndrom (POS). International wird üblicherweise von Attention Deficit/Hyperactivity Disorder (ADHD), bzw. Attention Deficit Disorder (ADD) gesprochen.

Das Aufmerksamkeitsdefizit-Hyperaktivitätssyndrom ist nach derzeitigem Stand ein multifaktoriell bedingtes Störungsbild mit einer erblichen Disposition, welche die Ausbildung der Krankheit begünstigt. Auf neurobiologischer Ebene wird es unter anderem als striatofrontale Dysfunktion erklärt. Für den Verlauf und die individuelle Ausprägung spielen daneben psychosoziale Faktoren und Umweltbedingungen eine wichtige Rolle.

Betroffene und ihre Angehörigen stehen meist unter erheblichem Druck. Versagen in Schule oder Beruf und die Entwicklung von weiteren psychischen Störungen sind häufig. Die Behandlung richtet sich nach dem Schweregrad, den jeweiligen Symptomen sowie dem Alter des Betroffenen. Wegen der Komplexität der Störung wird angestrebt, verschiedene Behandlungsansätze zu einer auf den Patienten und sein soziales Umfeld zugeschnittenen Therapie zu kombinieren.

Die Prävalenz von ADHS im Erwachsenenalter wird mit 1,3 % bis 4,7 % angegeben (Quelle: Deutsches Ärzteblatt Ausgabe 37 vom 10. September 2004). Zwischen 30 % und 70 % der ADHS-betroffenen Jugendlichen behalten die Störung auch im Erwachsenenalter bei (Persistenz); hierzu ist die Forschung aber noch nicht abgeschlossen. Im Erwachsenenalter nimmt Hyperaktivität einen veränderten Charakter an, indem sie sich als erhöhte innere Unruhe auswirkt. ADHS-Betroffene zeigen häufig verschiedene andere psychische Störungen z. B. Depressionen, Angststörungen und Störungen des Selbstbildes und Selbstwertgefühls, sowie soziale Phobien. Bei Frauen werden auch Essstörungen beobachtet. Betroffene beiderlei Geschlechts können Bulimie als Begleiterkrankung entwickeln. ADHS im Erwachsenenalter ist seit 1995 bekannt und seit 2003 auch in Deutschland anerkannt.

Zur medikamentösen Behandlung der ADHS werden in erster Linie Stimulanzien eingesetzt, welche den Dopaminstoffwechsel im Gehirn beeinflussen. Dazu gehören Methylphenidat und Amphetaminderivate (D-L Amphetamin), die etwa seit Mitte des letzten Jahrhunderts verwendet werden. Etwa 70 % der Betroffenen sprechen darauf an. Weiterhin können auch auf den Dopamin- oder Noradrenalinhaushalt wirkende Antidepressiva zur Behandlung eingesetzt werden.

Die heute zur Therapie des Aufmerksamkeitsdefizit-Hyperaktivitätssyndroms häufig eingesetzte Substanz Methylphenidat hemmt die Wiederaufnahme von Dopamin und Noradrenalin in den Präsynapsen und erhöht so deren Konzentration im synaptischen Spalt. Dies führt zu einem erhöhten Signalaufkommen am Rezeptor und unter anderem zu einer Erhöhung des Sympathikotonus. In geringem Maße sorgt Methylphenidat für die Freisetzung von Katecholaminen, die Erhöhung der Dopaminkonzentration nach Gabe von Methylphenidat wird aber in erster Linie durch Wiederaufnahmehemmung erreicht.

Der selektive Noradrenalin- und Dopamin- (geringfügig auch Serotonin-) Wiederaufnahmehemmer (NDRI) Bupropion kann auch erfolgreich bei ADHS verwendet werden, wobei die Substanz dafür keine Zulassung besitzt und an Patienten unter 18 Jahren nicht auf Wirksamkeit und Sicherheit geprüft wurde.

Pflanzen der Gattung *Sideritis*, die zur Familie der Lippenblütler (Lamiaceae) gehören, finden im Mittelmeerraum traditionell in der Volksmedizin bei entzündlichen Erkrankungen Anwendung. Die botanische Zuordnung der traditionell als "Bergtee" verwendeten *Sideritis*-Species erweist sich jedoch als sehr kompliziert. Sie haben je nach Region unterschiedlichste Namen und zum Teil nur lokale Bedeutung. In vielen Fällen pflückt und trocknet die Landbevölkerung *Sideritis-*Pflanzen an den Berghängen zum Eigengebrauch. Der aromatische Tee ist dann oft aus mehreren *Sideritis*-Arten zusammengesetzt. Bekannt sind etwa 150 *Sideritis*-Arten, die weltweit, aber hauptsächlich im Mittelmeerraum beheimatet sind. Alleine in der Türkei gibt es 46 und in Spanien 45 Arten *Sideritis,* die vielfach endemisch sind. Neben ihrer Artenvielfalt ist allen gemeinsam, dass sie zur Familie der Lippenblütler (Lamiaceae) gehören und leicht mit Salbei verwechselt werden. Neuere Arbeiten aus den letzten Jahren konnten nachweisen, dass verschiedene *Sideritis*-Arten antibakterielle, antioxidative, analgetische und entzündungshemmende Wirkung haben. Als Inhaltsstoffe wurden bisher Mono-, Di-, und Triterpene, Flavonoide und andere (z.B. Koffeinsäure) gefunden.

In der Druckschrift "Yusuf Öztürk et al.; Effects of Extracts of Certain Sideritis Species on Swimming Performance of Mice; in: Phytotherapy Research, Band 10, Seiten 70 bis 73 (1996)" wird beschrieben, dass *Sideritis*-Spezies als sedierendes und auch stimulierendes Heilmittel eingesetzt werden. Eine in der Druckschrift aus den Versuchsergebnissen abgeleitete Wirkung auf das ZNS der Versuchstiere hat jedoch nichts mit einer Wirkung auf die speziellen Neurotransmitter Noradrenalin und/oder Dopamin (und damit zu einer noradrenergen und/oder dopaminergen Wirkung) zu tun und ist erst recht kein Indiz für eine Wirkung als Noradrenalin-Wiederauf nahmehemmer, Dopamin-Wiederaufnahmehemmer oder Noradrenalin-Dopamin-Wiederauf nahmehemmer.

Überraschend wurde nun gefunden, dass Extrakte aus getrockneten Pflanzen der Gattung *Sideritis* in Noradrenalin-Wiederaufnahmeversuchen und Dopamin-Wiederaufnahmeversuchen mit Synaptosomen aus Rattencortex stark hemmende Eigenschaften auf die Wiederaufnahme von Noradrenalin und Dopamin hatten. Es wurde auch gefunden, dass das Trinken von aus *Sideritis-*Arten hergestelltem Kräutertee einen deutlichen Behandlungseffekt bei ADHS-Patienten zeigte.

Die Erfindung betrifft somit die Verwendung von Pflanzen der Gattung *Sideritis* und/oder von Teilen dieser Pflanzen und/oder von Extrakten dieser Pflanzen und/oder Pflanzenteile zur Behandlung von ADHS.

Die Erfindung betrifft auch die Verwendung von Pflanzen der Gattung *Sideritis* und/oder von Teilen dieser Pflanzen und/oder von Extrakten dieser Pflanzen und/oder Pflanzenteile zur Herstellung eines Arzneimittels zur Behandlung von ADHS.

Die Erfindung wird nachfolgend anhand bevorzugter Ausführungsformen und einzelner Beispiele, die ebenfalls bevorzugte Ausführungsformen betreffen, erläutert.

In bevorzugten Verwendungen gemäß der Erfindung kommen Pflanzenteile von *Sideritis,* z.B. Blätter, Stengel und Blüten, für die vorgenannten Zwecke zur Anwendung. In weiteren bevorzugten Ausführungsformen der Erfindung kommen Extrakte von Pflanzen und/oder Pflanzenteilen von *Sideritis* zur Anwendung. Alternativ dazu ist es auch möglich, Pflanzenteile und Extrakte von *Sideritis* zusammen zu verwenden.

Beispiele für *Sideritis*-Arten, die bei der vorliegenden Erfindung als Pflanzen der Gattung *Sideritis* enthalten sein können, sind *Sideritis syriaca* und *Sideritis scardica*.

Wenn Extrakte von *Sideritis* verwendet werden, sind dies beispielsweise alkoholisch-wässrige Extrakte. Alkohol bedeutet in diesem Zusammenhang niedere (C₁- bis C₄)- Alkohole. Das Volumenverhältnis der Alkohol-Wasser-Mischung für die Gewinnung der Extrakte aus *Sideritis* kann in weiten Grenzen schwanken. Es liegt besonders bevorzugt bei 99:1 bis 1:99 Vol.-%, noch weiter bevorzugt bei 65:35 bis 35:65 Vol.-%, am meisten bevorzugt bei 55:45 bis 45:55 Vol.-% (bezogen auf das Verhältnis Alkohol : Wasser in der gesamten, zur Extraktion verwendeten Mischung).

Als Extraktionsmittel können jedoch auch andere Mischungen, beispielsweise Mischungen von Wasser mit organischen Lösungsmitteln, die ebenfalls bevorzugt in den oben angegebenen Verhältnissen vorliegen, oder auch reine Lösungsmittel verwendet werden. Infrage kommen Wasser, Dimethylsulfoxid (DMSO), Mischungen von Kohlenwasserstoffen wie beispielsweise die unter dem Begriff "Petrolether" bekannten niedrig siedenden Benzin-Fraktionen (überwiegend Pentane und Hexane als Komponenten), halogenierte Kohlenwasserstoffe, insbesondere chlorierte Kohlenwasserstoffe, beispielsweise Mischungen aus Methylenchlorid und Chloroform sowie Ester niederer (C₁- bis C₄-) Alkohole mit niederen (C₁- bis C₄-) Carbonsäuren, beispielsweise Essigester (bevorzugt Essigsäureethylester).

Extraktionsmittel kann auch CO₂ sein. Eine CO₂-Extraktion kann beispielsweise (jedoch nicht ausschließlich) bei überkritischen Bedingungen erfolgen, was den Vorteil hat, für die zu extrahierenden Pflanzen der Gattung *Sideritis* besonders schonend zu sein.

In weiter bevorzugten Ausführungsformen der Erfindung werden die mittels der vorstehend beispielhaft genannten Extraktionsmittel hergestellten Extrakte nicht als solche verwendet, sondern in Form von Fraktionen, die aus den genannten Extrakten gewonnen wurden. Dies kann auf beliebenden, dem Fachmann als solches bekannten Wegen geschehen und bedarf daher an dieser Stelle keiner weiteren näheren Erläuterung.

Bevorzugt kommen die Pflanzen von *Sideritis* in Form der vorstehend genannten Extrakte zur Verwendung. Es ist jedoch auch möglich, sie in getrockneter Form zu verwenden.

Weiter können in anderen bevorzugten Ausführungsformen gemäß der Erfindung die Pflanzen der Gattung *Sideritis* und/oder deren Teile und/oder deren Extrakte und/oder aus solchen Extrakten hergestellte Fraktionen einzeln oder in Kombination miteinander in Kombination mit anderen Pflanzen in Form von Pflanzenteilen und/oder in Form von Extrakten solcher anderer Pflanzen verwendet werden. Genauso ist auch erfindungsgemäß bevorzugt, die Pflanzen der Gattung *Sideritis* und/oder deren Teile und/oder deren Extrakte und/oder aus solchen Extrakten hergestellte Fraktionen einzeln oder in Kombination mit chemisch-synthetischen Stoffen, z.B. nicht-selektive Monoaminwiederaufnahmehemmer (NSMRIs), selektive Serotoninwiederaufnahmehemmer (SSRIs), selektive Noradrenalinwiederaufnahmehemmer (NARIs), Serotonin-Noradrenalin-Wiederaufnahmehemmer SNRIs), Dopaminwiederaufnahmehemmer (DRIs), selektive Noradrenalin-Dopamin-Wiederaufnahmehemmer (NDRIs), MAO-Hemmer und andere, zu verwenden; auch im letztgenannten Fall können in einer bevorzugten Ausführungsform andere Pflanzen in Form von Pflanzenteilen und/oder Extrakten solcher Pflanzen verwendet werden.

Die erfindungsgemäße Verwendung gemäß den vorstehend beschriebenen Ausführungsformen kann eine Verwendung in Form einer pharmazeutischen Zubereitung sein. Als solche kann sie allgemein übliche und dem Fachmann als solche bekannte Darreichungsformen annehmen, die eine bestimmte Konzentration an *Sideritis*-Komponenten in einer Form enthalten, die einer Verabreichung mit einem bestimmten Plan (einmal täglich eine Dosis-Einheit oder dreimal täglich eine Dosis-Einheit, um nur zwei Beispiele zu nennen) folgt. Die Darreichungsform richtet sich in dem Fachmann bekannter Weise nach dem Verabreichungsweg (oral, intravenös, intramuskulär, nasal) und kann eine feste, halbfeste, flüssige, nebelartige, gasförmige oder andere die Verabreichung auf dem gewünschten Weg erlaubende Form sein.

Daneben kann die erfindungsgemäße Verwendung ebenfalls in einer Verwendung als Nahrungsergänzungsmittel bestehen.

Die folgenden Beispiele einer bevorzugten Ausführungsform erläutern die Erfindung.

### Neurotransmitterwiederaufnahmeexperimente:

### Versuchsdurchführung:

### Extraktherstellung:

*Sideritis*-Pflanzenteile wurden mit Methanol oder Wasser (1g *Sideritis* pro 10 ml Lösungsmittel) über 2 Stunden bei Siedetemperatur unter Rückfluss extrahiert. Der erhaltene Extrakt wurde filtriert und dann mit Hilfe eines Rotationsverdampfers zur Trockene abgezogen. Die Extrakte wurden aus verschiedenen kommerziell erhältlichen "Bergtee"-Proben hergestellt. Folgende Quellen standen zum Bezug der *Sideritis*-Proben zur Verfügung:
1. Griechischer Bergtee, Tee Gschwendner, Meckenheim, Filiale Freiburg (*Sideritis scardica*)
   Extrakt 1, Methanolextrakt
2. Griechischer Bergtee, Salameh Co., Seitingen-Oberflacht
   Extrakt 2, Methanolextrakt
   Extrakt 3, Wasserextrakt
3. Malotira-Mountain Tea, Cretan traditional products "Melissa", Agios Nikolaos, Griechenland.
   Extrakt 4, Methanolextrakt.

### Dopamin- und Noradrenalin-Wiederaufnahmeexperimente:

Männliche Wistar-Ratten (250-300 g) wurden einzeln in einem klimatisierten Raum (21 +/- 2 °C) mit 12 h hell/dunkel-Zyklus gehalten. Die Tiere erhielten Futter und Wasser ad libitum.

Die Tiere wurden unter CO₂-Narkose dekapitiert und das Gehirn schnell entnommen. Der Cortex wurde auf Eis herauspräpariert. Das Cortexgewebe wurde in 10 Volumen eiskalter 0,32 M Saccharoselösung (mit 2,5 mM HEPES, pH 7,4) mit einem Potter-Elvehjem Glas/Teflon Homogenisator unter Eiskühlung homogenisiert und das entstandene Homogenat 10 min bei 900*g und 4 °C zentrifugiert. Der Überstand wurde entnommen, in Eppendorf-Hütchen überführt und 10 min bei 11.000*g (4°C) zentrifugiert. Die so präparierten Synaptosomen wurden bis zur weiteren Verwendung auf Eis gelagert.

Das verwendete Puffersystem war physiologischer Puffer pH 7,4 (140 mM NaCl, 5 mM KCl, 1,8 mM CaCl₂, 0,8 mM MgSO₄, 11 mM Glucose, 25 mM HEPES, 0,6 mM Ascorbinsäure) mit 50 µM Pargylin (Sigma-Aldrich, Taufkirchen) als MAO-Hemmer.

10 µl einer DMSO-Lösung der Extrakte aus *Sideritis species* in verschiedenen Konzentrationen wurden zusammen mit 180 µl Puffer in eine Vertiefung einer 96-well Filterplatte (Millipore MultiScreenFB) gegeben. Für Vergleichsexperimente zur Bestimmung der unspezifischen Bindung wurden der Noradrenalin- und Dopaminwiederaufnahmehemmer Nomifensin (Sigma-Aldrich, Taufkirchen) in einer Konzentration von 10 µM eingesetzt.

Die auf Eis gelagerten Synaptosomen wurden in Puffer resuspendiert und jeweils 50 µl jedem Ansatz zugegeben. Der Ansatz wurde bei Raumtemperatur 10 min geschüttelt. Anschließend wurden schnell 10 µl [³H]-Neurotransmitterlösung zugegeben (Endkonzentration 20 nM Noradrenalin bzw. 10 nM Dopamin; Perkin-Elmer Life Sciences, Rodgau) und der Ansatz 15 min bei 37 °C unter schütteln inkubiert.

Die Reaktion wurde durch Absaugen und mehrfaches Spülen mit Puffer beendet. Die Radioaktivität im Filter wurde nach Herausstanzen aus der Platte durch Szintillationsmessungen bestimmt. Die spezifische Aufnahme ist definiert als die gesamte aufgenommene Radioaktivität abzüglich der Radioaktivität, die in Gegenwart von 10 µM Nomifensin noch bindet.

Die Auswertung der Versuche führte zu folgenden spezifischen Wiederaufnahmeraten:

| ***Noradrenalin:*** | | | | |
|---|---|---|---|---|
| | Extrakt 1 | Extrakt 2 | Extrakt 3 | Extrakt 4 |
| Kontrolle | 1,00 ± 0,12 | 1,00 ± 0,17 | 1,00 ± 0,17 | 1,00 ± 0,17 |
| | | | | |
| *Sideritis* | | | | |
| 5 µg/ml | 0,96 ± 0,11 | 0,71 ± 0,32 | 0,78 ± 0,16 | 0,62 ± 0,20 |
| 50 µg/ml | 0,48 ± 0,08 | 0,46 ± 0,26 | 0,22 ± 0,07 | 0,17 ± 0,26 |
| 500 µg/ml | 0,17 ± 0,02 | -0,38 ± 0,05 | -0,22 ± 0,13 | -0,52 ± 0,11 |
| | | | | |
| EC₅₀: | 42,3 µg/ml | 81,2 µg/ml | 30,6 µg/ml | 36,9 µg/ml |
| | [31,8; 56,4] | [54,5; 120,8] | [25,1; 37,5] | [27,3; 49,9] |
| | | | | |

| ***Dopamin:*** | | | | |
|---|---|---|---|---|
| | Extrakt 1 | Extrakt 2 | Extrakt 3 | Extrakt 4 |
| Kontrolle | 1,00 ± 0,11 | 1,00 ± 0,04 | 1,00 ± 0,04 | 1,00 ± 0,04 |
| | | | | |
| *Sideritis* | | | | |
| 5 µg/ml | 0,85 ± 0,04 | 1,02 ± 0,10 | 0,98 ± 0,05 | 1,02 ± 0,12 |
| 50 µg/ml | 0,51 ± 0,07 | 0,73 ± 0,05 | 0,65 ± 0,23 | 0,79 ± 0,07 |
| 500 µg/ml | 0,16 ± 0,02 | -0,13 ± 0,07 | 0,43 ± 0,06 | -0,22 ± 0,01 |
| | | | | |
| EC₅₀: | 37,0 µg/ml | 293,2 µg/ml | 45,5 µg/ml | 57,8 µg/ml |
| | [27,5; 49,8] | [230,9; 372,3] | [31,4; 66,0] | [39,2; 85,2] |

| | | | | |
|---|---|---|---|---|
| Vergleichsexperimente mit Methylphenidat ergaben eine EC₅₀ von 0,9 µM [0,7; 1,3] für die Hemmung der Dopaminwiederaufnahme. (angegeben ist jeweils der Mittelwert ± 95%-Konfidenzintervall aus 8 Versuchen). | | | | |

### Fallbeschreibungen:

### 1. ADHS im Kindesalter vom hyperkinetisch-impulsiven Typ mit Störung des Sozialverhaltens (ICD-10: F90.1)

13 jährige Probandin. In der Schule und im häuslichen Alltag leichte bis mittelgradige Störung von Konzentration, Aufmerksamkeit und Auffassungsgabe, dabei starke motivationale Schwankungen, vor sich herschieben von unangenehmen Tätigkeiten. Hört im Gespräch mit der Mutter nicht zu, vergisst Aufträge, verliert Gegenstände, starke psychomotorische Anspannung mit Unruhe und hoher Reizbarkeit, ausgeprägte Impulskontrollstörung mit verbalagressiven Entgleisungen und gelegentlichen körperlichen Übergriffen, oppositionelles Verhalten und Verweigerung gegenüber den Erziehungsberechtigten. In Spannungszuständen selbstverletzendes Verhalten durch Ritzen der Haut mit Glasscherben und scharfkantigen Blechstücken, launisch-dramatisches Temperament, ausgeprägte Affektlabilität und starke Stimmungschwankungen, Angst vor dem Alleinsein, innere Unruhe. Im formalen Gedankengang geordnet bis zum Teil leicht beschleunigt, Rededrang, Gedankensprünge. Mehrfach die Woche konflikthafte Zuspitzung mit Neigung, sich in Auseinandersetzung und Diskussion zu verwickeln.

Interventionsphase mit 1 l Bergtee (3 g *Sideritis scardica* pro Liter, nicht spezies-charakterisiert). In der ersten Woche für die Eltern deutliche Minderung der wahrgenommenen Anspannung der Probandin, das Mädchen kann besser zuhören, unterbricht weniger und kann bei Meinungsverschiedenheiten sachlich bleiben ohne krisenhafte Entwicklung und Auseinandersetzung. Die Probandin erledigt ihre Hausaufgaben sorgfältiger mit weniger Flüchtigkeitsfehlern und ist im häuslichen Umfeld innerhalb der Familie sozial verträglicher nach Einschätzung der Angehörigen. Ab der vierten Woche Nachlassen der Compliance, die Probandin lehnt die tägliche regelmäßige Aufnahme der Teezubereitung aus geschmacklichen Gründen ab. Für eine andere Darreichungsform wäre die Probandin zugänglich gewesen, da sie selbst die Verbesserung der Verhaltensstörung bemerkte und ihr Leidensdruck durch die verbesserte Anpassungsfähigkeit sich reduziert hatte.

GCI (Global Clinical Impression) (Baseline) 6; GCI (Woche 3) 4-5.

Nach Absetzen des Bergtees stellte sich innerhalb von zwei Tagen wieder der Ausgangszustand ein.

### 2. ADHS im Erwachsenenalter vom hyperkinetisch-impulsiven Typ (ICD-10: F 90.0)

35 jähriger männlicher Proband. In der Baseline zeigte sich psychopathologisch eine mittelgradige Beeinträchtigung von Aufmerksamkeit, Konzentration und Auffassungsgabe, hohe Interferenzierung bei tonischer Alertness, hohe Ablenkbarkeit bei geteilter Aufmerksamkeit, ständiges Verlegen und Vergessen von Gegenständen, Rededrang, starke motorische und innere Unruhe mit ständigem Wippen der Extremitäten und mangelnder Fähigkeit still zu sitzen. Ausgeprägte emotionale Instabilität mit schweren reaktiven Stimmungswechseln, ausgeprägte assoziative Lockerung des formalen Gedankengangs mit Gedankensprüngen und gelegentlicher Inkohärenz, ständiges dazwischenreden und herausplatzen im Gespräch, in den Schilderungen weitschweifig und umständlich. Reduzierte Stresstoleranz mit intermittierend explosivem und distanzlosem Verhalten, geringes Selbstvertrauen und Selbstwertproblematik.

Intervention mit Bergtee (21 pro Tag, etwa 3 g *Sideritis scardica* pro Liter), Konsum über den Tag verteilt. Bereits in der ersten Woche der Anwendung zeigten sich für den Probanden, das soziale Umfeld und die Untersucher signifikante Verbesserungen der Konzentrations-, Aufmerksamkeits- und Auffassungsgabe mit Minderung des Problemverhaltens. Der Proband kann jetzt im Gespräch aufmerksam folgen und zuhören, gibt Sachverhalte prägnant und strukturiert wieder, lässt das Gegenüber ausreden und hält den Gesprächsfaden. Psychomotorisch deutliche Entspannung mit verbesserter Impulskontrolle und Stresstoleranz, Stimmungsstabilisierung und - aufhellung. Anhaltender Effekt über mehrere Monate mit täglichem Konsum der oben genannten Bergteemenge. Bei Auslassversuch innerhalb von zwei Wochen wieder Zunahme der Psychopathologie ohne Erreichen des Ausgangsschweregrades.

GCI (Baseline) 5; GCI (Woche 3) 3.

## Patentansprüche

1. Pflanzen der Gattung *Sideritis* und/oder Teile dieser Pflanzen und/oder Extrakte dieser Pflanzen und/oder Pflanzenteile zur Verwendung in der Behandlung des Aufmerksamkeitsdefizit-Hyperaktivitätssyndroms.

2. Verwendung von Pflanzen der Gattung *Sideritis* und/oder von Teilen dieser Pflanzen und/oder von Extrakten dieser Pflanzen und/oder Pflanzenteile zur Herstellung eines Arzneimittels zur Behandlung des Aufmerksamkeitsdefizit-Hyperaktivitätssyndroms.

3. Pflanzen der Gattung *Sideritis* und/oder Teile dieser Pflanzen und/oder Extrakte dieser Pflanzen und/oder Pflanzenteile zur Verwendung nach Anspruch 1 oder Verwendung nach Anspruch 2, **dadurch gekennzeichnet, dass** als Extrakte Wasser-Extrakte und/oder Extrakte mit organischen Lösungsmitteln und/oder Extrakte mit Mischungen aus Wasser und einem oder mehreren organischen Lösungsmitteln und/oder CO₂-Extrakte verwendet werden.

4. Pflanzen der Gattung *Sideritis* und/oder Teile dieser Pflanzen und/oder Extrakte dieser Pflanzen und/oder Pflanzenteile oder Verwendung nach Anspruch 3, **dadurch gekennzeichnet, dass** als organische Lösungsmittel Alkohole, insbesondere C₁-C₄-Alkohole, Dimethylsulfoxid (DMSO), Petrolether, halogenierte Kohlenwasserstoffe, vorzugsweise chlorierte Kohlenwasserstoffe, insbesondere Methylenchlorid und/oder Chloroform, Ester niederer (C₁-C₄-) Alkohole mit niederen (C₁-C₄-) Carbonsäuren, insbesondere Essigester, besonders bevorzugt Essigsäureethylester, eingesetzt werden.

5. Pflanzen der Gattung *Sideritis* und/oder Teile dieser Pflanzen und/oder Extrakte dieser Pflanzen und/oder Pflanzenteile oder Verwendung nach Anspruch 3 oder 4, **dadurch gekennzeichnet, dass** Fraktionen der Extrakte, in flüssiger oder getrockneter Form, verwendet werden.

6. Pflanzen der Gattung *Sideritis* und/oder Teile dieser Pflanzen und/oder Extrakte dieser Pflanzen und/oder Pflanzenteile oder Verwendung nach einem der Ansprüche 3 bis 5, **dadurch gekennzeichnet, dass** das Mischungsverhältnis von Wasser zu organischem Lösungsmittel 99:1 bis 1:99 Vol.-%, bevorzugt 65:35 bis 35:65 Vol.-% und besonders bevorzugt 55:45 bis 45:55 Vol.-% beträgt.

7. Pflanzen der Gattung *Sideritis* und/oder Teile dieser Pflanzen und/oder Extrakte dieser Pflanzen und/oder Pflanzenteile oder Verwendung nach einem der Ansprüche 1 bis 6, **dadurch gekennzeichnet, dass** die Pflanzen der Gattung *Sideritis* und/oder die Teile dieser Pflanzen und/oder die Extrakte dieser Pflanzen und/oder Pflanzenteile in Kombination mit anderen Pflanzen in Form von Pflanzenteilen und/oder Extrakten und/oder in Kombination mit chemisch-synthetischen Stoffen verwendet werden.

8. Pflanzen der Gattung *Sideritis* und/oder Teile dieser Pflanzen und/oder Extrakte dieser Pflanzen und/oder Pflanzenteile oder Verwendung nach einem der Ansprüche 1 bis 7 in Form einer pharmazeutischen Zubereitung und/oder in Form eines Nahrungsergänzungsmittels.

## Claims

1. Plants of the genus *Sideritis* and/or parts of these plants and/or extracts of these plants and/or parts of plants for use in the treatment of an attention deficit disorder.

2. Use of plants of the genus *Sideritis* and/or parts of these plants and/or extracts of these plants and/or parts of plants for preparing a medicament for the treatment of an attention deficit disorder.

3. Plants of the genus *Sideritis* and/or parts of these plants and/or extracts of these plants and/or parts of plants for a use according to claim 1 or a use according to claim 2, **characterized in that** water extracts and/or extracts with organic solvents and/or Extracts with mixtures of water and one or more organic solvents and/or CO₂ extracts are used as extracts.

4. Plants of the genus *Sideritis* and/or parts of these plants and/or extracts of these plants and/or parts of plants or use according to claim 3, **characterized in that** alcohols, in particular C₁-C₄ alcohols, dimethylsulfoxide (DMSO), petrol ether, halogenated hydrocarbons, preferably chlorinated hydrocarbons, in particular methylene chloride and/or chloroform, esters of lower (C₁-C₄) alcohols with lower (C₁-C₄) carboxylic acids, in particular acetic acid esters, particularly preferred acetic acid ethyl ester, are employed as organic solvents.

5. Plants of the genus *Sideritis* and/or parts of these plants and/or extracts of these plants and/or parts of plants or use according to claim 3 or 4, **characterized in that** fractions of the extracts are used, in liquid or dried form.

6. Plants of the genus *Sideritis* and/or parts of these plants and/or extracts of these plants and/or parts of plants or use according to any of claims 3 to 5, **characterized in that** the mixing ratio of water and organic solvent is 99:1 to 1:99 % by volume, preferably 65:35 to 35:65 % by volume and particularly preferred 55:45 to 45:55 % by volume.

7. Plants of the genus *Sideritis* and/or parts of these plants and/or extracts of these plants and/or parts of plants or use according to any of claims 1 to 6, **characterized in that** the plants of the genus *Sideritis* and/or parts of these plants and/or extracts of these plants and/or parts of plants are used in combination with other plants in form of parts of plants and/or extracts and/or in combination with chemically synthesized substances.

8. Plants of the genus *Sideritis* and/or parts of these plants and/or extracts of these plants and/or parts of plants or use according to any of claims 1 to 7 in form of a pharmaceutical preparation and/or in form of a dietary supplement.

## Revendications

1. Plantes appartenant au genre *Sideritis* et/ou parties desdites plantes et/ou extraits desdites plantes et/ou parties de plantes, destinés à l'utilisation dans le traitement du trouble du déficit de l'attention/hyperactivité.

2. Utilisation de plantes appartenant au genre *Sideritis* et/ou de parties desdites plantes et/ou d'extraits desdites plantes et/ou de parties de plantes pour la préparation d'un médicament destiné au traitement du trouble du déficit de l'attention/hyperactivité.

3. Plantes appartenant au genre Sideritis et/ou parties desdites plantes et/ou extraits desdites plantes et/ou parties de plantes, destinés à l'utilisation selon la revendication 1 ou à l'utilisation selon la revendication 2, **caractérisés en ce que** l'on utilise, en tant qu'extraits, des extraits à l'eau et/ou des extraits aux solvants organiques et/ou des extraits aux mélanges d'eau avec un ou plusieurs solvants organiques et/ou des extraits au CO₂.

4. Plantes appartenant au genre *Sideritis* et/ou parties desdites plantes et/ou extraits desdites plantes et/ou parties de plantes, destinés à l'utilisation selon la revendication 3, **caractérisés en ce que** l'on utilise, en tant que solvants organiques, des alcools, notamment des alcools en C₁-C₄, du diméthylsulfoxyde (DMSO), de l'éther de pétrole, des hydrocarbures halogénés, préférentiellement des hydrocarbures chlorés, notamment du dichlorométhane et/ou du chloroforme, des esters que les alcools à courte chaîne (C₁-C₄) forment avec des acides carboxyliques à courte chaîne (C₁-C₄), notamment des esters de l'acide acétique, s'agissant avec une préférence particulière de l'acétate d'éthyle.

5. Plantes appartenant au genre *Sideritis* et/ou parties desdites plantes et/ou extraits desdites plantes et/ou parties de plantes, destinés à une utilisation selon les revendications 3 ou 4, **caractérisés en ce que** l'on utilise des fractions desdits extraits, sous forme liquide ou séchée.

6. Plantes appartenant au genre *Sideritis* et/ou parties desdites plantes et/ou extraits desdites plantes et/ou parties de plantes, destinés à une utilisation selon l'une des revendications 3 à 5, **caractérisés en ce que** le rapport du mélange entre l'eau et le solvant organique est compris 99 : 1 et 1 : 99 % en volume, de préférence entre 65 : 35 et 35 : 65 % en volume et, avec une préférence particulière, entre 55 : 45 et 45 : 55 % en volume.

7. Plantes appartenant au genre *Sideritis* et/ou parties desdites plantes et/ou extraits desdites plantes et/ou parties de plantes, destinés à une utilisation selon l'une des revendications 1 à 6, **caractérisés en ce que** lesdits plantes appartenant au genre *Sideritis* et/ou parties desdites plantes et/ou extraits desdites plantes et/ou parties de plantes sont utilisés en combinaison avec d'autres plantes sous forme de parties de plantes et/ou d'extraits et/ou en combinaison avec des substances issues d'une synthèse chimique.

8. Plantes appartenant au genre *Sideritis* et/ou parties desdites plantes et/ou extraits desdites plantes et/ou parties de plantes ou utilisation selon l'une des revendications 1 à 7 sous forme d'une préparation pharmaceutique et/ou sous forme d'un complément alimentaire.
